# NEW EUROPEAN PATENT SPECIFICATION

(11) **EP 3 448 370 B2**
(45) Date of publication and mention of the opposition decision: **12.07.2023**
(45) Mention of the grant of the patent: 21.10.2020
(21) Application number: 17730926.7
(22) Date of filing: 27.04.2017
(51) Int. Cl.: A61K 31/047, A61K 31/519, A61K 31/593, A61K 33/24, A61K 36/61, A61P 5/26, A61P 43/00

(54) **COMPOSITION FOR THE TREATMENT OF POLYCYSTIC OVARY SYNDROME**
ZUSAMMENSETZUNG ZUR VERWENDUNG BEI DER BEHANDLUNG VON POLYZYSTISCHEM OVARIALSYNDROM
COMPOSITION POUR LE TRAITEMENT DU SYNDROME DES OVAIRES POLYKYSTIQUES

(30) Priority: 29.04.2016 IT UA20163025
(43) Date of publication of application: 06.03.2019
(73) Proprietor: Sochim International SpA, 20010 Cornaredo (MI) (IT)
(72) Inventor: EIGENMANN, Carlo, 20010 Cornaredo (MI) (IT); DE LEO, Vincenzo, 20010 Cornaredo (MI) (IT)
(74) Representative: Villa, Livia
(86) International application number: PCT/IB2017/052430
(87) International publication number: WO 2017/187375

(56) References cited:
- EP-A1- 3 165 229
- EP-A1- 3 165 229
- WO-A1-2013/016741
- WO-A1-2013/016742
- Anonymous: "Combining science and nature AntaGolin... - Insulin resistance, raising awareness through our journey | Facebook", , 15 November 2013 (2013-11-15), XP055331781, Retrieved from the Internet: URL:https://www.facebook.com/InsulinResist anceRaisingAwarenessThroughOurJourney/post s/334934786647039 [retrieved on 2017-01-03]
- Anonymous: "AntaGolin combats insulin resistance through multiple pathways of action", , 3 January 2017 (2017-01-03), XP055331782, Retrieved from the Internet: URL:http://mnilifestyle.co.za/products/ant agolin/ [retrieved on 2017-01-03]

## Description

The present invention relates to a composition for the treatment of polycystic ovary syndrome, as well as the endocrine-gynecological and metabolic disorders associated therewith. The composition of the invention comprises inositol and banaba extract as the only active agents in the treatment of polycystic ovary syndrome, wherein inositol is present in an amount higher than banaba extract.

### STATE OF THE ART

The polycystic ovary syndrome (PCOS) is a multifactorial syndrome that affects 10 percent of women of reproductive age. The presence of a polycystic ovary is essentially related to a problem characterized by an ovarian dysfunction and menstrual disorders, often associated with some clinical and biochemical signs of hyperandrogenism, hirsutism, acne and ultrasound scanned ovarian cysts.

The problem underlying polycystic ovary is insulin resistance.

At the onset of insulin resistance, the body activates a compensation mechanism by increasing the release of insulin, and in these cases hyperinsulinism arises, i.e. a production of high levels of the hormone insulin in the blood.

The pathophysiology has established a connection between the onset of insulin resistance and the deficiency of essential elements, such as copper, magnesium, zinc, manganese, chromium and calcium, but also vitamin D deficiencies or inflammation.

Insulin stimulates the production of both estrogen and testosterone, and the higher the insulin, the greater the amount of hormones. In women in normal conditions, testosterone is then converted into estrogens, and only a minimal part remains in the bloodstream. Conversely, in the polycystic ovary syndrome, testosterone production is so high that the majority fails to undergo this transformation. But the excess testosterone in the bloodstream activates the pituitary gland to produce another luteinizing hormone, which in turn stimulates the hormone production in the ovaries. This hormone production that cannot be successful because the hormonal cycle is not complete.

It is well understood how this mechanism is self-maintained. At each glycaemia sudden change, the body reacts with homeostatic mechanisms thus recreating the vicious circle. The pancreas is unable to counteract this continuous demand for insulin. If stress continues, then cortisol arises, which is a counter-regulator hormone of insulin that completely blocks the functions thereof. Also cortisol has a hyperglycemizing action, induces insulin resistance while reducing the uptake and utilization of glucose by the muscle and the adipose tissue.

This mechanism clearly disregulates all hormones and alters the rhythm of the menstrual cycle in women, resulting in short cycles, anovulatory cycles, and infertility.

Drugs are able to decrease the ovarian androgen overproduction (object achieved also by surgical intervention in case of no response to medication). Some drugs are aimed at increasing the secretion of FHS hormone; others block the action of estrogens at the level of the membrane receptors.

Along with medical therapy and a suitable diet, it may be desirable to associate the uptake of products that improve the general condition of patients and reduce symptoms and insulin resistance typical of the polycystic ovary syndrome.

It is therefore the object of the present invention to provide an effective solution in the treatment of polycystic ovary syndrome.

### SUMMARY OF THE INVENTION

Said object is achieved by a composition comprising inositol and banaba extract, as set forth in claim 1.

In another aspect, the present invention relates to said composition for use in the treatment of polycystic ovary syndrome.

As will be clear from the following detailed description and from the working examples provided by way of illustration, the composition of the invention has surprisingly allowed drastically reducing the dosage of inositol to be taken in a day. This reduction on a daily basis can in fact reach 50% and more. Also, while generally the first results with inositol as such are observed after at least 3 months of treatment, often after 6 months, with the composition of the invention the first results can be observed just after one month of treatment, thus demonstrating a significantly faster action.

The characteristics and advantages of the present invention will appear more clearly from the following detailed description and from the embodiments shown in the examples.

### DETAILED DESCRIPTION OF THE INVENTION

The invention therefore relates to a composition comprising inositol and banaba extract as the only active agents in the treatment of polycystic ovary syndrome, wherein inositol is present in an amount higher than banaba extract.

For the purposes of the present invention, the term "inositol" means myo-inositol, scyllo-inositol, muco-inositol, D-chiro-inositol, L-chiro-inositol, neo-inositol, epi-inositol, cis-inositol, or a mixture thereof.

For the purposes of the present invention, the term "banaba extract" means a dry extract of the leaves of *Lagerstroemia speciosa L.,* a tree native to Southeast Asia, particularly the Philippines, Malaysia, Taiwan, Thailand and India.

This dry extract powder is commercially available and is obtained by hydro-alcoholic extraction, preferably by ethanol/water extraction.

In particular, this extract can be obtained by shredding the leaves of *Lagerstroemia speciosa L.,* ethanol extraction, filtration and concentration, addition of water, filtration and drying.

Alternately, this extract can be obtained by water extraction.

In preferred embodiments, said inositol is myo-inositol.

In preferred embodiments of the invention, inositol and banaba extract are in a weight ratio of 2:1 to 300:1. Preferably, inositol and banaba extract are in a weight ratio of 10:1 to 200:1, more preferably 20:1 to 150:1.

As can be seen from the weight ratios above, a minimal amount of banaba extract with respect to the amount of inositol is sufficient to achieve a synergistic effect that allows reducing the daily dosage of inositol by at least 50%.

In some embodiments, the composition comprises up to 98% by weight of inositol, more preferably up to 75% by weight.

For the purposes of the present invention, unless otherwise stated, "% by weight" means % by weight on the weight of the composition of the invention.

In other embodiments, the composition comprises up to 10% by weight of banaba extract, more preferably up to 5% by weight.

In preferred embodiments, inositol and banaba extract are the only active ingredients present in the composition of the invention, where "active" means active in the treatment of polycystic ovary syndrome, the other components being pharmaceutically acceptable adjuvants or excipients.

Suitable adjuvants preferably are vitamin, pharmaceutically acceptable chromium salt, folic acid, catechins, coenzyme Q10, cinnamon extract or a mixture thereof.

Suitable vitamins are vitamin A, vitamin B1, vitamin B2, vitamin B6, vitamin B3, vitamin B9, vitamin B12, vitamin C, vitamin D, vitamin E, and vitamin K.

In other embodiments, the composition of the invention further comprises vitamin D. Preferably, the composition comprises up to 0.05% by weight of vitamin D, more preferably up to 0.005% by weight.

"Vitamin D" is a fat-soluble prohormone group consisting of vitamin D1, vitamin D2, vitamin D3, vitamin D4, vitamin D5 or a mixture thereof.

Preferably, vitamin D is vitamin D3. Studies in fact identify vitamin D3, or cholecalciferol, as the form of vitamin D that is more effective in ensuring correct concentrations of this nutrient in the blood. The integration of vitamin D3 is recommended in patients with polycystic ovarian syndrome, because insulin resistance and obesity are associated with a deficiency of this vitamin. In particular, the following was observed:
- a correlation between low vitamin D levels and endocrine/metabolic imbalances associated with PCOS,
- a direct effect on insulin sensitivity, because vitamin D stimulates insulin receptor expression on insulin-targeted peripheral cells,
- an indirect effect on insulin sensitivity, as insulin secretion and insulin sensitivity are calcium-dependent processes; vitamin D influences them indirectly by regulating calcium absorption and homeostasis,
- a systemic anti-inflammatory effect: systemic inflammation is known for its important role in the pathogenesis of insulin resistance and type II diabetes; vitamin D acts positively in several ways, such as by modulating the expression of proinflammatory cytokines.

In other embodiments, the composition of the invention further comprises a pharmaceutically acceptable chromium salt. Suitable chromium salts are chromium chloride, chromium sulfate, chromium nitrate, chromium lactate trihydrate, chromium nicotinate, chromium picolinate, chromium citrate, or a mixture thereof. In preferred embodiments, said pharmaceutically acceptable chromium salt is chromium picolinate, as it is particularly bioavailable.

Preferably, the composition of the invention comprises up to 0.05% by weight of pharmaceutically acceptable chromium salts, more preferably up to 0.005% by weight.

The addition of chromium in the form of pharmaceutically acceptable salts is advisable in the treatment of polycystic ovary syndrome, since it is believed that said salt enhances the effect of insulin, as well as the inositol/banaba extract association of the invention, with different mechanisms of action, namely:
- enhancement of the insulin-receptor binding,
- increase in the number of receptors,
- increased phosphorylation of receptors and hence sensitivity to the hormone,
- belonging to the LMWCr complex (chromoduline), which is supposed to amplify the insulin receptor response to insulin.

In other embodiments, the composition of the invention further comprises folic acid. Preferably, the composition comprises up to 0.5% by weight of folic acid, more preferably up to 0.05% by weight.

Folic acid integration improves the insulin cellular sensitivity.

In particular, there is evidence that subjects with PCOS have homocysteine values higher than the standard; folic acid contrasts hyperomocysteinemia, a recognized cardiovascular risk factor (pro-inflammatory, pro-atherogenic and pro-thrombotic effect); it is also believed that high values of homocysteine may compromise the embryo implant and interfere with the correct vascularization of the endometrium, thus increasing the risk of spontaneous abortion. Moreover, folic acid intake is recommended as nutrition supplementation in fertile women for the prevention of neural tube defects.

In preferred embodiments, the composition of the invention further comprises vitamin D and a pharmaceutically acceptable chromium salt.

In preferred embodiments, the composition of the invention further comprises vitamin D and folic acid.

In preferred embodiments, the composition of the invention further comprises folic acid and a pharmaceutically acceptable chromium salt.

In particularly preferred embodiments, the composition of the invention further comprises vitamin D and a pharmaceutically acceptable chromium salt and folic acid.

In further embodiments, the composition of the invention further comprises vitamin D, a pharmaceutically acceptable chromium salt, folic acid or a mixture thereof.

It is to be understood that the preferred aspects identified for the single components, namely vitamin D, pharmaceutically acceptable chromium salts and folic acid, are to be deemed as similarly preferred in the embodiments that comprise them in combination with one another.

In other embodiments, the composition of the invention further comprises at least one catechin.

Said at least one catechin is epigallocatechin gallate (EGCG), epigallocatechin (EGC), epicatechin gallate (ECG), epicatechin (EC), or a mixture thereof.

Preferably, said at least one catechin is epigallocatechin gallate (EGCG).

Catechins, in addition to exhibiting good antioxidant activity and improving glucose metabolism, have also demonstrated a good bioavailability that allows, in particular to EGCG, reaching tissue levels around 8-10% of the blood levels in the major organs (heart, brain, liver, uterus, etc.).

According to a preferred embodiment, said at least one catechin is in the form of a green tea extract titrated at min. 10%.

In some embodiments, the composition comprises up to 10% by weight of catechin, more preferably up to 5% by weight.

In other embodiments, the composition of the invention further comprises Coenzyme Q10.

Coenzyme Q10 (CoQ10) provides the energy necessary for cellular functions and is known to act as an antioxidant. It also improves glycemic control by improving insulin secretion, which is without adverse effects.

In some embodiments, the composition comprises up to 10% by weight of Coenzyme Q10, more preferably up to 5% by weight.

In other embodiments, the composition of the invention further comprises cinnamon extract.

The cinnamon extract induces a significant reduction in insulin resistance.

In some embodiments, the composition comprises up to 80% by weight of cinnamon extract, more preferably up to 50% by weight.

In preferred embodiments, the composition of the invention further comprises at least one catechin and Coenzyme Q10.

In preferred embodiments, the composition of the invention further comprises at least one catechin and cinnamon extract.

In preferred embodiments, the composition of the invention further comprises Coenzyme Q10 and cinnamon extract.

In particularly preferred embodiments, the composition of the invention further comprises at least one catechin, Coenzyme Q10 and cinnamon extract.

In further preferred embodiments, the composition of the invention further comprises epigallocatechin gallate (EGCG), epigallocatechin (EGC), epicatechin gallate (ECG), epicatechin (EC), Coenzyme Q10, cinnamon extract, or a mixture thereof.

It is to be understood that the preferred aspects identified for the single components, namely at least one catechin, coenzyme Q10 and cinnamon extract, are to be deemed as similarly preferred in the embodiments that comprise them in combination with one another.

In other embodiments, the composition of the invention consists essentially of inositol and banaba extract. The expression "consists essentially of" means that inositol and banaba extract are the only ingredients present in the composition of the invention, active in the treatment of polycystic ovary syndrome, the other components being pharmaceutically acceptable adjuvants or excipients that do not interfere with the action of inositol and banaba extract.

In other embodiments, the composition of the invention does not contain berberine.

In further embodiments, the composition of the invention consists of inositol, banaba extract, pharmaceutically acceptable adjuvants and excipients.

It is to be understood that all aspects identified as preferred and advantageous for the composition of the invention should be deemed as similarly preferred and advantageous also for these embodiments.

In preferred embodiments, the composition of the invention is in a unit dosage form. Preferably, said unit dosage form comprises 1-10,000 mg inositol, more preferably 100-8,000 mg.

In particularly preferred embodiments, said unit dosage form includes 500-5,000 mg inositol.

Preferably, said unit dosage form comprises 0.1-100 mg banaba extract, more preferably 1-75 mg.

In particularly preferred embodiments, said unit dosage form includes 10-50 mg banaba extract.

Preferably, said unit dosage form further comprises up to 1,000 U.I. of vitamin D, more preferably 50-800 U.I..

Preferably, said unit dosage form further comprises 1-500 µg of a pharmaceutically acceptable chromium salt, more preferably 1-100 µg.

Preferably, said unit dosage form further comprises 10-1,500 µg folic acid, more preferably 50-1,000 µg.

In preferred embodiments, said unit dosage form includes 0.1-100 mg banaba extract, 1-10,000 mg inositol, up to 1000 U.I. of vitamin D, 1-500 µg of a pharmaceutically acceptable chromium salt, and 10-1,500 µg of folic acid.

In particularly preferred embodiments, said unit dosage form includes 10-50 mg banaba extract, 800-5,000 mg inositol, 50-800 U.I. of vitamin D, 1-100 µg of a pharmaceutically acceptable chromium salt, and 50-1.000 µg of folic acid.

Further preferred are compositions and unit dosage forms comprising:

| | |
|---|---|
| myo-inositol | 1,000 mg |
| banaba extract | 48 mg |
| Vitamin D3 | 400 U.I. (10 µg) |
| chromium picolinate | 40 µg |
| folic acid | 400 µg |

or

| | |
|---|---|
| myo-inositol | 2,000 mg |
| banaba extract | 48 mg |
| Vitamin D3 | 400 U.I. (10 µg) |
| chromium picolinate | 40 µg |
| folic acid | 400 µg |

or

| | |
|---|---|
| myo-inositol | 4,000 mg |
| banaba extract | 48 mg |
| Vitamin D3 | 400 U.I. (10 µg) |
| chromium picolinate | 40 µg |
| folic acid | 400 µg |

or

| | |
|---|---|
| myo-inositol | 1,000 mg |
| banaba extract | 32 mg |
| Vitamin D3 | 400 U.I. (10 µg) |
| chromium picolinate | 40 µg |
| folic acid | 400 µg |

or

| | |
|---|---|
| myo-inositol | 2,000 mg |
| banaba extract | 32 mg |
| Vitamin D3 | 400 U.I. (10 µg) |
| chromium picolinate | 40 µg |
| folic acid | 400 µg |

or

| | |
|---|---|
| myo-inositol | 4,000 mg |
| banaba extract | 32 mg |
| Vitamin D3 | 400 U.I. (10 µg) |
| chromium picolinate | 40 µg |
| folic acid | 400 µg |

or

| | |
|---|---|
| myo-inositol | 1,000 mg |
| banaba extract | 16 mg |
| Vitamin D3 | 400 U.I. (10 µg) |
| chromium picolinate | 40 µg |
| folic acid | 400 µg |

or

| | |
|---|---|
| myo-inositol | 2,000 mg |
| banaba extract | 16 mg |
| Vitamin D3 | 400 U.I. (10 µg) |
| chromium picolinate | 40 µg |
| folic acid | 400 µg |

or

| | |
|---|---|
| myo-inositol | 4,000 mg |
| banaba extract | 16 mg |
| Vitamin D3 | 400 U.I. (10 µg) |
| chromium picolinate | 40 µg |
| folic acid | 400 µg |

It is to be understood that the preferred aspects identified for the single components are to be deemed as similarly preferred in the unit dosage form embodiments as described above.

The composition of the invention, also in a unit dosage form, may further comprise pharmaceutically acceptable excipients. By "excipient" it is meant a compound or a mixture thereof suitable for the use in a formulation for the treatment of polycystic ovary syndrome or conditions associated therewith. For example, an excipient for use in a pharmaceutical formulation generally should not cause an adverse reaction in a subject, nor it should significantly inhibit the efficacy of the composition.

Suitable excipients are acidifying agents, acidity correctors, anti-agglomerants, antioxidants, fillers, resistance agents, gelling agents, coating agents, modified starches, sequestering agents, thickeners, sweeteners, thinners, solvents, disaggregating agents, glidants, dyes, binders, lubricants, stabilizers, adsorbents, preservatives, wetting agents, flavors, film-forming substances, emulsifiers, wetting agents, release retardants and mixtures thereof.

Preferably, said excipients are starch, modified starch, cellulose, modified cellulose, microcrystalline cellulose, sodium carboxymethylcellulose, pectin, tragacanth, mannitol, dicalcium phosphate, xanthan gum, carrageenan, sodium alginate, guar gum, maltodextrin, silicon dioxide, or mixtures thereof.

The composition of the present invention can be prepared by methods known in the art. In fact, for oral administration, the components may, for example, be mixed with one or more excipients, enclosed in soft gel capsules or made in a solid form, such as tablet, mini-tablet, micro-tablet, granule, micro-granule, pellets, multiparticulate, micronized particulate, powder, or in the form of solution, emulsion, gel, vial, drops or sprays.

In another aspect, the present invention relates to the above composition for use in the treatment of the polycystic ovary syndrome. As said, "treatment of the polycystic ovary syndrome" refers to both the treatment and the prevention not only of the polycystic ovary syndrome but also of the endocrine-gynecological and metabolic disorders related thereto, such as alterations of the menstrual cycle, hyperandrogenism, insulin resistance and pre-diabetic conditions. In fact, the composition of the invention has surprisingly allowed drastically reducing the dosage of inositol to be taken in a day. Moreover, with the composition of the invention, the first results can be observed even just after one month of treatment, thus demonstrating an advantageously high rate of action.

A composition according to the invention was in fact tested, comprising inositol and banaba extract as the only active components in the treatment of polycystic ovary syndrome. For this test, women with PCOS were enrolled (Rotterdam criteria-based diagnosis), with insulin resistance demonstrated by the Homa index (>2.5). The enrolled patients were all treated with the composition of the invention 1 packet/day. Before and after one month of treatment, patients enrolled were subjected to a glucose loading curve (75g) for the determination of glucose and insulin levels every 30 min for 2 hours (i.e. at 0, 30, 60, 90 and 120 minutes).

The study showed that after only one month of treatment with the composition of the invention, significant changes in glucose and insulin levels were obtained, demonstrating how the synergy between inositol and banaba extract (in particular myo-inositol and dry extract of banaba leaves) is able to influence insulin resistance in such a short time (1 month) that is one of the main causes behind the onset of PCOS. Improvements in BMI, length of the menstrual cycle, and hormone dosages were also observed.

The composition of the invention may also advantageously be used as a coadjuvant in pregnancy-seeking support, especially in women with physiological fertility reduction/over 35, as well as as an adjuvant for induction of ovulation in medically assisted procreation programs.

The composition of the invention may be administered via oral, buccal, or sublingual route.

Preferably, the composition of the invention is administered via oral route.

More preferably, the composition of the invention is administered at least once a day.

In preferred embodiments, the composition of the invention is administered once or twice a day, in a unit dosage form as described above.

It should be understood that all the possible combinations of preferred aspects of the components of the composition, as above reported, are to be deemed as hereby disclosed. It should be understood also that all the aspects identified as preferred and advantageous for the composition and its components are to be deemed as similarly preferred and advantageous also for the preparations and uses thereof.

Below are working examples of the present invention provided for illustrative purposes.

### EXAMPLES

### Example 1.

The following composition was prepared:

| | |
|---|---|
| - myo-inositol | 1,000 mg |
| - banaba extract | 48 mg |
| - Vitamin D3 | 400 U.I. (10 µg) |
| - chromium picolinate | 40 µg |
| - folic acid | 400 µg |

### Example 2.

The following composition was prepared:

| | |
|---|---|
| - myo-inositol | 2,000 mg |
| - banaba extract | 48 mg |
| - Vitamin D3 | 400 U.I. (10 µg) |
| chromium picolinate | 40 µg |
| folic acid | 400 µg |

### Example 3.

The following composition was prepared:

| | |
|---|---|
| myo-inositol | 4,000 mg |
| banaba extract | 48 mg |
| Vitamin D3 | 400 U.I. (10 µg) |
| chromium picolinate | 40 µg |
| folic acid | 400 µg |

### Example 4.

The following composition was prepared:

| | |
|---|---|
| myo-inositol | 1,000 mg |
| banaba extract | 32 mg |
| Vitamin D3 | 400 U.I. (10 µg) |
| chromium picolinate | 40 µg |
| folic acid | 400 µg |

### Example 5.

The following composition was prepared:

| | |
|---|---|
| myo-inositol | 2,000 mg |
| banaba extract | 32 mg |
| Vitamin D3 | 400 U.I. (10 µg) |
| chromium picolinate | 40 µg |
| folic acid | 400 µg |

### Example 6.

The following composition was prepared:

| | |
|---|---|
| myo-inositol | 4,000 mg |
| banaba extract | 32 mg |
| Vitamin D3 | 400 U.I. (10 µg) |
| chromium picolinate | 40 µg |
| folic acid | 400 µg |

### Example 7.

The following composition was prepared:

| | |
|---|---|
| myo-inositol | 1,000 mg |
| banaba extract | 16 mg |
| Vitamin D3 | 400 U.I. (10 µg) |
| chromium picolinate | 40 µg |
| folic acid | 400 µg |

### Example 8.

The following composition was prepared:

| | |
|---|---|
| - myo-inositol | 2,000 mg |
| - banaba extract | 16 mg |
| - Vitamin D3 | 400 U.I. (10 µg) |
| - chromium picolinate | 40 µg |
| - folic acid | 400 µg |

### Example 9.

The following composition was prepared:

| | |
|---|---|
| - myo-inositol | 4,000 mg |
| - banaba extract | 16 mg |
| - Vitamin D3 | 400 U.I. (10 µg) |
| - chromium picolinate | 40 µg |
| - folic acid | 400 µg |

## Claims

1. A composition comprising inositol and banaba extract, wherein inositol and banaba extract are the only ingredients, present in the composition, to be active in the treatment of polycystic ovary syndrome, wherein inositol is present in an amount higher than banaba extract.

2. The composition of claim 1 wherein inositol and banaba extract are in a weight ratio of 2:1 to 300:1.

3. The composition of claim 2 wherein inositol and banaba extract are in a weight ratio of 10:1 to 200:1, preferably 20:1 to 150:1.

4. The composition of any one of claims 1-3 4, comprising up to 98% by weight of inositol, more preferably up to 75% by weight.

5. The composition of any one of claims 1-4 5-, comprising up to 10% by weight of banaba extract, more preferably up to 5% by weight.

6. The composition of any one of claims 1-5 6, further comprising Vitamin D, a pharmaceutically acceptable salt of chromium, folic acid, or a mixture thereof.

7. The composition of any one of claims 1-6 further comprising epigallocatechin gallate (EGCG), epigallocatechin (EGC), epicatechin gallate (ECG), epicatechin (EC), Coenzyme Q10, cinnamon extract, or a mixture thereof.

8. The composition of any one of claims 1-7 in a unit dosage form comprising 1-10000 mg of inositol, more preferably 100-8000 mg.

9. The composition of claim 8 9, in a unit dosage form comprising 0.1-100 mg of banaba extract, more preferably 1-75 mg.

10. The composition of any one of claims 1-7 comprising:
| | |
|---|---|
| - myo-inositol | 1000 mg |
| - banaba extract | 48 mg |
| - Vitamin D3 | 400 UI (10 µg) |
| - chromium picolinate | 40 µg |
| - folic acid | 400 µg |
or
| | |
|---|---|
| - myo-inositol | 2000 mg |
| - banaba extract | 48 mg |
| - Vitamin D3 | 400 UI (10 µg) |
| - chromium picolinate | 40 µg |
| - folic acid | 400 µg |
or
| | |
|---|---|
| - myo-inositol | 4000 mg |
| - banaba extract | 48 mg |
| - Vitamin D3 | 400 UI (10 µg) |
| - chromium picolinate | 40 µg |
| - folic acid | 400 µg |
or
| | |
|---|---|
| - myo-inositol | 1000 mg |
| - banaba extract | 32 mg |
| - Vitamin D3 | 400 UI (10 µg) |
| - chromium picolinate | 40 µg |
| - folic acid | 400 µg |
or
| | |
|---|---|
| - myo-inositol | 2000 mg |
| - banaba extract | 32 mg |
| - Vitamin D3 | 400 UI (10 µg) |
| - chromium picolinate | 40 µg |
| - folic acid | 400 µg |
or
| | |
|---|---|
| - myo-inositol | 4000 mg |
| - banaba extract | 32 mg |
| - Vitamin D3 | 400 UI (10 µg) |
| - chromium picolinate | 40 µg |
| - folic acid | 400 µg |
or
| | |
|---|---|
| - myo-inositol | 1000 mg |
| - banaba extract | 16 mg |
| - Vitamin D3 | 400 UI (10 µg) |
| - chromium picolinate | 40 µg |
| - folic acid | 400 µg |
or
| | |
|---|---|
| - myo-inositol | 2000 mg |
| - banaba extract | 16 mg |
| - Vitamin D3 | 400 UI (10 µg) |
| - chromium picolinate | 40 µg |
| - folic acid | 400 µg |
or
| | |
|---|---|
| - myo-inositol | 4000 mg |
| - banaba extract | 16 mg |
| - Vitamin D3 | 400 UI (10 µg) |
| - chromium picolinate | 40 µg |
| - folic acid | 400 µg |

11. The composition of any one of claims 1-10 for use in the treatment of polycystic ovary syndrome.

## Patentansprüche

1. Zusammensetzung, umfassend Inositol und Banaba-Extrakt, wobei Inositol und Banaba-Extrakt die einzigen Bestandteile sind, die in der Zusammensetzung vorhanden sind, um bei der Behandlung von polyzystischem Ovar-Syndrom aktiv zu sein, wobei Inositol in einer höheren Menge als Banaba-Extrakt vorhanden ist.

2. Zusammensetzung nach Anspruch 1, wobei Inositol und Banaba-Extrakt in einem Gewichtsverhältnis von 2:1 bis 300:1 sind.

3. Zusammensetzung nach Anspruch 2, wobei Inositol und Banaba-Extrakt in einem Gewichtsverhältnis von 10:1 bis 200:1, vorzugsweise 20:1 bis 150:1, sind.

4. Zusammensetzung nach einem der Ansprüche 1-3, umfassend bis zu 98 Gewichtsprozent Inositol, vorzugsweise bis zu 75 Gewichtsprozent.

5. Zusammensetzung nach einem der Ansprüche 1-4, umfassend bis zu 10 Gewichtsprozent Banaba-Extrakt, vorzugsweise bis zu 5 Gewichtsprozent.

6. Zusammensetzung nach einem der Ansprüche 1-5, ferner umfassend Vitamin D, ein pharmazeutisch annehmbares Chromsalz, Folsäure oder ein Gemisch davon.

7. Zusammensetzung nach einem der Ansprüche 1-6, ferner umfassend Epigallocatechingallat (EGCG), Epigallocatechin (EGC), Epicatechingallat (ECG), Epicatechin (EC), Coenzym Q10, Zimtextrakt oder ein Gemisch davon.

8. Zusammensetzung nach einem der Ansprüche 1-7 in einer Einheitsdosisform, umfassend 1-10000 mg Inositol, bevorzugter 100-8000 mg.

9. Zusammensetzung nach Anspruch 8 in einer Einheitsdosisform, umfassend 0,1-100 mg Banaba-Extrakt, vorzugsweise 1-75 mg.

10. Zusammensetzung nach einem der Ansprüche 1-7, umfassend:
| | |
|---|---|
| - Myo-Inositol | 1000 mg |
| - Banaba-Extrakt | 48 mg |
| Vitamin D3 | 400 UI (10 µg) |
| - Chrompicolinat | 40 µg |
| Folsäure | 400 µg |
oder
| | |
|---|---|
| - Myo-Inositol | 2000 mg |
| - Banaba-Extrakt | 48 mg |
| Vitamin D3 | 400 UI (10 µg) |
| - Chrompicolinat | 40 µg |
| Folsäure | 400 µg |
oder
| | |
|---|---|
| - Myo-Inositol | 4000 mg |
| - Banaba-Extrakt | 48 mg |
| Vitamin D3 | 400 UI (10 µg) |
| - Chrompicolinat | 40 µg |
| Folsäure | 400 µg |
oder
| | |
|---|---|
| - Myo-Inositol | 1000 mg |
| - Banaba-Extrakt | 32 mg |
| Vitamin D3 | 400 UI (10 µg) |
| - Chrompicolinat | 40 µg |
| Folsäure | 400 µg |
oder
| | |
|---|---|
| - Myo-Inositol | 2000 mg |
| - Banaba-Extrakt | 32 mg |
| Vitamin D3 | 400 UI (10 µg) |
| - Chrompicolinat | 40 µg |
| Folsäure | 400 µg |
oder
| | |
|---|---|
| - Myo-Inositol | 4000 mg |
| - Banaba-Extrakt | 32 mg |
| Vitamin D3 | 400 UI (10 µg) |
| - Chrompicolinat | 40 µg |
| Folsäure | 400 µg |
oder
| | |
|---|---|
| - Myo-Inositol | 1000 mg |
| - Banaba-Extrakt | 16 mg |
| Vitamin D3 | 400 UI (10 µg) |
| - Chrompicolinat | 40 µg |
| Folsäure | 400 µg |
oder
| | |
|---|---|
| - Myo-Inositol | 2000 mg |
| - Banaba-Extrakt | 16 mg |
| Vitamin D3 | 400 UI (10 µg) |
| - Chrompicolinat | 40 µg |
| Folsäure | 400 µg |
oder
| | |
|---|---|
| - Myo-Inositol | 4000 mg |
| - Banaba-Extrakt | 16 mg |
| Vitamin D3 | 400 UI (10 µg) |
| - Chrompicolinat | 40 µg |
| Folsäure | 400 µg |

11. Zusammensetzung nach einem der Ansprüche 1-10 zur Verwendung bei der Behandlung von polyzystischem Ovar-Syndrom.

## Revendications

1. Composition comprenant de l'inositol et un extrait de banaba, ledit inositol et ledit extrait de banaba étant les seuls ingrédients, présents dans la composition, à être actifs dans le traitement du syndrome des ovaires polykystiques, ledit inositol étant présent en une quantité supérieure à l'extrait de banaba.

2. Composition selon la revendication 1, ledit inositol et ledit extrait de banaba étant dans un rapport massique de 2:1 à 300:1.

3. Composition selon la revendication 2, ledit inositol et ledit extrait de banaba étant dans un rapport massique de 10:1 à 200:1, de préférence 20:1 à 150:1.

4. Composition selon l'une quelconque des revendications 1 à 3, comprenant jusqu'à 98 % en poids d'inositol, plus préférablement jusqu'à 75 % en poids.

5. Composition selon l'une quelconque des revendications 1 à 4, comprenant jusqu'à 10 % en poids d'extrait de banaba, plus préférablement jusqu'à 5 % en poids.

6. Composition selon l'une quelconque des revendications 1 à 5, comprenant en outre de la vitamine D, un sel de chrome pharmaceutiquement acceptable, de l'acide folique ou un mélange de ceux-ci.

7. Composition selon l'une quelconque des revendications 1 à 6, comprenant en outre du gallate d'épigallocatéchine (EGCG), de l'épigallocatéchine (EGC), du gallate d'épicatéchine (ECG), de l'épicatéchine (EC), de la coenzyme Q10, de l'extrait de cannelle, ou un mélange de ceux-ci.

8. Composition selon l'une quelconque des revendications 1 à 7, sous une forme posologique unitaire comprenant 1 à 10000 mg d'inositol, plus préférablement 100 à 8000 mg.

9. Composition selon la revendication 8, sous une forme posologique unitaire comprenant 0,1 à 100 mg d'extrait de banaba, plus préférablement 1 à 75 mg.

10. Composition selon l'une quelconque des revendications 1 à 7, comprenant:
| | |
|---|---|
| - du myo-inositol | 1000 mg |
| - de l'extrait de banaba | 48 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 2000 mg |
| - de l'extrait de banaba | 48 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 4000 mg |
| - de l'extrait de banaba | 48 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 1000 mg |
| - de l'extrait de banaba | 32 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 2000 mg |
| - de l'extrait de banaba | 32 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 4000 mg |
| - de l'extrait de banaba | 32 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 1000 mg |
| - de l'extrait de banaba | 16 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 2000 mg |
| - de l'extrait de banaba | 16 mg |
| - de la vitamine D3 | 400 UI (10 µg) |
| - du picolinate de chrome | 40 µg |
| - de l'acide folique | 400 µg |
ou
| | |
|---|---|
| - du myo-inositol | 4000 mg |
| - de l'extrait de banaba | 16 mg |
| - de la vitamine D3 | 400 UI (10 µg) |
| - du picolinate de chrome | 40 µg |
| - de l'acide folique | 400 µg. |

11. Composition selon l'une quelconque des revendications 1 à 10 pour utilisation dans le traitement du syndrome des ovaires polykystiques.
